# EUROPEAN PATENT APPLICATION

(11) **EP 2 979 689 A1**
(43) Date of publication of application: **03.02.2016**
(21) Application number: 14178883.6
(22) Date of filing: 29.07.2014
(51) Int. Cl.: A61K 9/12, A61K 31/535

(54) **Composition for an eye drop and delivery system therefor**

(71) Applicant: Sygene Technologies, Palos Heights, IL 60463 (US)
(72) Inventor: Grant, Calvin, Oak Park, IL Illinois 60302 (US)
(74) Representative: Eke, Philippa Dianne

(57) **Abstract**

The present application describes an ophthalmic composition including a foaming agent, adapted for topical administration to the eye from an applicator capable of dispensing a foam.

## Description

### Field of the invention

The present invention relates to an ophthalmic composition for topical application to the eye and to a system for the delivery of the same.

### Background to the invention

Most ophthalmic compositions for application to the eye come in the form of an eye drop or an ointment. Most ophthalmic ointment bases comprise a mixture of mineral oil and white petrolatum and have a melting point close to body temperature; however they can blur vision as they remain viscous and are not easily removed by tear fluid. Ointments are generally used at night, in addition to eye drops which are used during the day. In eye drops, the medicament is in aqueous form and delivered by an eye dropper. Despite most people's familiarity with the eye dropper, many find it difficult to use and often apply too little medication by misplacing the drop, or too much medication by distributing more than one drop at time. Recent studies have found that 61% to 90% of patients with glaucoma, one of the largest eye drop user groups, are unable to correctly use the standard dropper.

Commonly, a dropper delivers 50-70 microliters of medication in a single drop. However, the normal volume of tears in the eye is estimated to be just 7 microliters, and if blinking occurs, the eye can only hold up to 10 microliters without spillage. Even if the drop lands in the in correct position, the natural tendency of the eye is to immediately reduce the volume to 7 microliters. The result of this is that 86% to 90% of all eye medication today is wasted. Innovations that have been made to improve this design have often just attempted to improve the delivery of the drop to the eye; products such as Xal-Ease™, VersiDoser™ and Visine® Pure Tears Single Drop dispenser are examples of this. These methods still do not solve the problem that the liquid drop is an inefficient method of delivery of medication to the eye.

### Summary of the invention

The present invention provides a liquid ophthalmic composition comprising a foaming agent. The foaming agent allows the liquid to form a foam when mixed with a gas, and typically increases the viscosity of the solution, thereby increasing stability, lubricating properties and bioavailabilty. The composition is delivered by a foam dispensing pump container or other device that is capable of dispensing a foam.

Thus the formulation of a topical eye medication is changed so that it is capable of being dispensed as a foam rather than as a liquid. By foaming the eye medication several benefits can be obtained, one of the most important of which is the reduction of wasted medication. This is achieved because the foam can reach a sufficient volume to form a drop and fall into the eye without exceeding the amount of fluid that they eye can actually hold. In other words, in using the present invention the actual volume of liquid medication that is administered per drop can match what the eye can actually hold, since the majority of the volume of the drop is comprised of gas. The reduction in volume dispensed to the eye will also reduce the rate at which the eye replaces the tear film. This will allow the medication to remain in contact with the eye for a longer amount of time and thus increase the bioavailability of the drug.

By foaming the drop, the drop also becomes larger and easier to apply to the eye. Another significant advantage of the present invention is that the gas that is used to create the foam can be used to augment the effectiveness of the drop. The foaming drop also has the added benefits of comfort of use, ease of application, and higher patient compliance. The invention described here is a more effective and more efficient method of delivery of medication to the eye.

### Detailed description of the invention

The present invention provides a liquid ophthalmic composition including a foaming agent, adapted for topical administration to the eye from an applicator capable of dispensing a foam. The foaming agent enables a foam to form when the liquid ophthalmic composition is mixed with a gas.

The present invention also provides a topical ophthalmic composition delivery system comprising a liquid ophthalmic composition including a foaming agent, and an applicator capable of dispensing a foam.

The present invention further provides an ophthalmic foam containing a therapeutically active agent and a foaming agent.

The composition of the present invention allows the liquid ophthalmic composition to form a stable foam when mixed with a gas. This is achieved by the addition of one or more foaming agents. There is a variety of foaming agents from which to choose.

Suitable foaming agents include, but are not limited to:
Carbohydrates, for example cellulose derivatives, chitosan, cyclodextrins, gums, quince seed extract, starch, chemically modified starches, and sugars. Suitable cellulose derivatives include carboxymethylcellulose, cationic cellulose, hydroxyethyl carboxymethylcellulose, hydroxyethyl cellulose, hydroxypropyl cellulose, hydroxypropyl methylcellulose, methylcellulose, and methyl hydroxyethyl cellulose. Examples of gums include alginic acid, carrageenan, gellan gum, guar gum, cationic guars, hydroxypropyl guar gum, locust bean gum, sodium alginate, tragacanth gum, and xanthan gum. Suitable sugars include sucrose and trehalsoe;
Corneal penetration enhancers, such as surfactants, including detergents and non-ionic surfactants. Examples include laurocapram, hexamethylene lauramide, hexamethylene octanamide, and decylmethy sulfoxide;
Gelling agents, for example gelatin and agar;
Glycosaminoglycans, for example hyaluronic acids and salts;
Lipids, for example cetyl alcohol, stearyl alcohol, and stearic acid;
Proteins, for example albumins and sodium caseinate. Suitable albumins include bovine serum albumin, egg albumin, human serum albumin, and synthetic albumin;
Polyols, for example ethylene glycol, glycerol, and propylene glycol;
Synthetic polymers, for example amine-bearing polymers, carboxyvinyl polymers, polyacrylic acid, polyethylene glycol, polyvinyl acetate, polyvinyl alcohol, polyvinyl chloride, polyvinylidene chloride, and polyvinylpyrrolindone. Suitable polyethylene glycol varieties include PEG 1000, PEG 4000, PEG 6000, and PEG 8000.

In one embodiment, the foaming agent is one that is able to augment the effectiveness of the active ingredient. The majority of the foaming agents herein are viscosity-enhancing and augment the effectiveness of the active ingredient by increasing contact time to enhance bioavailability. The corneal penetration enhancers increase bioavailability by enhancing the permeability of the cornea to allow the active ingredients to reach their site of action.

Preferably, the foaming agent has properties such that a large reduction in volume between the foam and the liquid is obtained. Preferably the foaming agent generates a foam having a viscosity of between 1 and 10,000 MPAs at 25°C. For example, the foaming agent generates a foam having a viscosity of between 1 and 1,000 MPAs at 25°C, between 1 and 200 MPAs at 25°C, between 1 and 150 MPAs at 25°C, between 1 and 125 MPAs at 25°C, between 1 and 100 MPAs at 25°C, and especially between 1 and 50 MPAs at 25°C, such as between 1 and 40, 1 and 30, 1 and 20, 1 and 15, or 1 and 10 MPAs at 25°C. The foaming agent should be chosen to reduce the risk of eye irritation and to minimize any potential binding of the active ingredient, and preferably, it should not affect the stability of the solution.

Preferred foaming agents are, independently, cellulose derivatives, corneal penetration enhancers, and albumins, all as described above.

A preferred foaming agent is hydroxypropyl methyl cellulose. A solution containing between 0.25% and 5% of hydroxypropyl methylcellulose is particularly preferred. This has been proven to produce a foam that is capable of holding enough gas to result in a 90% reduction in volume of the medication distributed in a drop. In other words, the foam that is created is 90% gas and 10% liquid. Hydroxypropyl methylcellulose is especially preferred because it is already known to be a safe ingredient to be applied to the eye as it is currently used in many eye drops to lubricate the eye. Compared with water, which is a common solvent in topical eye medications, it also produces a more viscous solution which increases the retention time in the eye, thus giving the medication more time to be absorbed.

Another preferred foaming agent is an albumin. A solution containing between 1 % and 40%, or between 2% and 40%, or between 1 % and 25% of albumin is particularly preferred. Albumin has been found to be an excellent foaming agent capable of producing foams of the desired viscosity,

A non-ionic surfactant that is a known corneal penetration enhancer, such as laurocapram, is another preferred foaming agent, especially when the active ingredient is hydrophilic and would benefit from increased corneal absorption. The use of a penetration enhancer as the foaming component of the composition has the benefit of further lowering the amount of active ingredient required, as substances such as laurocapram have been shown to greatly increase corneal penetration for hydrophilic molecules. Examples of other enhancers suitable as foaming agents include, but are not limited to, hexamethylene lauramide, hexamethylene octanamide, and decylmethy sulfoxide.

In formulating the pharmaceutical composition of the present invention, the foaming agent is selected to enhance the active ingredient and thereby gain the greatest benefit.

Mucoadhesive polymers and in situ gelling excipients have also been found to further increase drug bioavailability, and may be included in the compositions of the present invention. Mucoadhesive polymers can be divided into two classes: hydrophilic polymers and hydrogels, and in situ gelling agents. Some of the foaming agents herein are also mucoadhesive polymers. For example, cellulose derivatives (methylcellulose, ethylcellulose, hydroxypropyl methylcellulose), sodium alginate, carrageenan, and xanthan gum are all hydrophilic polymers. Sodium alginate is an ion-activated in situ gelling agent. Thus, some foaming agents can also have mucoadhesive and in situ gelling effects. Mucoadhesive polymers and in situ gelling agents that do not primarily function as foaming agents may also be added to the formulation. Mucoadhesive polymers enhance drug bioavailability by increasing corneal contact time, allowing the drug more time to diffuse towards target tissues.

The liquid ophthalmic composition of the present invention may contain a therapeutically active agent in solution or suspension. Alternatively, or additionally, a therapeutically active agent may be contained in the gas which is mixed with the liquid to form a foam. Thus, in most applications of the present invention the active ingredient will be either dissolved or suspended in the liquid; however, the gas that is used to form the foam can also be therapeutic. This type of distribution could use medicinal gases such as hydrogen, carbon monoxide, hydrogen sulphide, xenon, ozone, and especially, nitrous oxide. When a gas is used to provide a medical benefit, it can be used in combination with an aqueous active ingredient or it can be used just with the foaming agent.

The composition of the present invention is for topical administration to the eye from an applicator capable of dispensing a foam.

Such topical administration may be achieved in various ways. The preferred method is by using as the applicator a foam dispensing pump container. As such, the present invention provides a topical ophthalmic composition delivery system comprising a liquid ophthalmic composition including a foaming agent, and a foam dispensing pump container.

Suitable pump containers include those of the types disclosed in U.S. Pat. No. 5,271,530, 5,429,279, and 5,337,929. The foaming pump described in U.S Pat. Application 0096412 is especially suitable due to its ability to function while inverted. Such containers may be produced in a size optimized for ophthalmic delivery and with a nozzle head resembling a typical eye dropper. These foaming mechanisms use air from the surrounding area but they could also be modified to use any type of gas.

When a foam dispensing pump container is used as the applicator, no propellant is required. Thus, in one embodiment, the composition of the present invention excludes a propellant.

The composition of the present invention is capable of being used in an aerosol device as well. When the composition is foamed using an aerosol device, any type of gas such as medicinal gases and inert gases may be used. When an aerosol is used the medication and or foaming agent is under pressure in liquid form along with the propellant. The use of an aerosol as the delivery method also has the advantage of reducing the need for a preservative. The composition can be formulated just as described above. The can of the aerosol container would preferably be of a smaller size to suit the dosage that is common in most eye drops and the nozzle which supplies the foam should be modified to make application to the eye easier. Another possible delivery method for this device is a pressurized gas canister with hose leading to an applicator that mixes the gas and fluid to create foam. This type of delivery system would be especially useful in the surgical environment. The methods of delivery described here is certainly not a complete list. The presented pharmaceutical composition can be used with any type of device that is capable of mixing a fluid and gas in order to produce a foam.

The ophthalmic foam of the present invention contains a foaming agent as described above and a therapeutically active agent.

A wide range of therapeutically active agents may be employed in the present invention. The nature of the therapeutic agent is not important, provided it is an agent suitable for application to the eye. Many such therapeutic agents are known, including for example:
acyclovir,antazoline,apraclonidine,atropine,azelastine,bacitracin, bepotastine besilate, betaxolol, bimatoprost, brimonidine tartrate, brinzolamide, bromfenac, carboxymethylcellulose, carteolol hydrochloride, ciprofloxacin hydrochloride, conium maculatum, cromolyn sodium, cyclopentolate, cyclosporine, dexamethasone, dextran, diclofenac, dipivefrin hydrochloride, dorzolamide hydrochloride, emedastine, erythromycin, euphrasia, famciclovir, fluorometholone, flurbiprofen, gatifloxacin, gentamicin, glycerin, gramicidin, hepar sulphuris calcareum, homatropine, hydroxypropylmethylcellulose, isoptoatropine sulfate, isoptohomatropine hydrobromide, ketorolac, ketotifenfumarate, latanoprost, levobunolol hydrochloride, levocabastine hydrochloride light mineral oil (also called mineral oil, paraffin), lodoxamide tromethamine, loteprednol etabonate, moxifloxacin hydrochloride, multivitamin, N-Acetyl-L-Carnosine, naphazoline, naphazoline hydrochloride, naphazolineHydrochloride, natrium muriaticum, nedocromil sodium sulfate, neomycin, nepafenac, norfloxacin, ofloxacin, olopatadine hydrochloride, oxymetazoline hydrochloride, pectin, pemirolast potassium, petrolatum (also called white petrolatum, white petroleum), pheniramine maleate, phenylephrine hydrochloride, pilocarpine hydrochloride, polyethyleneglycol (for example,polyethylene glycol 400), polymixin, polymyxin B sulfate, polysorbate 80, polyvinyl alcohol (PVA), povidone, povidone-iodine, prednisolone, prednisone,proparacaine hydrochloride, propyleneglycol, rimexolone, ruta graveolens, sodium chloride, sufacetamide sodium, soy lecithin, tetracaine, tetrahydrozoline hydrochloride, tetrahydrozoline hydrochloride, timolol maleate, tobramycin, trifluridine, trimethoprim, tropicamide, unoprostone isoproppyl, valacyclovir (also called valaciclovir), vacomycin, and zinc sulfate.

Also useful may be a recombinant DNA, antibody, protein, or biopharmaceutical for medicinal purposes.

In one embodiment of the present invention, the active ingredient is selected from bimatoprost, prednisolone and timolol, in particular, timolol.

The table below gives examples of medicaments using active ingredients such as the above, some of which contain two or more active ingredients. The present invention is of use with such medicaments.

**Table 1.**

| **Medicament** | **Active Ingredients** |
|---|---|
| Acular | ketorolac |
| Acyclovir | acyclovir |
| Adsorbocarpine | pilocarpine |
| Advanced Eye Relief Environmental | glycerin |
| Advanced Eye Relief Night Time | mineral oil, white petrolatum |
| Advanced Eye Relief Rejuvenation | glycerin |
| Ak-Dilate | phenylephrine |
| Ak-Pentolate | cyclopentolate |
| Akarpine | pilocarpine |
| Alamast | pemirolast |
| Alcaine | proparacaine |
| Almocarpine | pilocarpine |
| Alocril | nedocromil |
| Alomide | lodoxamide tromethamine |
| Alphagan | brimonidine tartrate |
| Alrex | loteprednol etabonate |
| Ami-Pilo | pilocarpine |
| Amistura P | pilocarpine |
| Astelin | azelastine |
| Atropine | isopto atropine |
| Atropisol | atropine |
| Azopt | brinzolamide |
| Bepreve | bepotastine besilate |
| Beta-pilocarpine hydrochloride | pilocarpine |
| Betagan | levobunolol |
| Betimol | timolol |
| Betoptic | betaxolol |
| Bion Tears | hydroxypropyl methylcellulose, dextran |
| Bleph-10 | sulfacetamide |
| Blephamide | Sodium sufacetamide, prednisolone |
| Blink Contacts | polyethylene glycol 400 |
| Carnosine | N-Acetyl-L-Carnosine |
| Chibroxin | norfloxacin |
| Ciloxan | ciprofloxacin hydrochloride |
| Clarymist Dwelle | polyvinyl alcohol, soy lecithin, povidone |
| Clear Eyes 7 Symptom Relief | hydroxypropyl methylcellulose, naphazoline HCL, polysorbate 80, zinc sulfate |
| Clear Eyes Contact Lens Relief | hydroxypropyl methylcellulose, glycerin |
| Clear Eyes Maximum Itchy Eye Relief | glycerin, naphazoline HCL, zinc sulfate |
| Clear Eyes Maximum Redness Relief | glycerin, naphazoline HCL, zinc sulfate |
| Clear Eyes Natural Tears | polyvinyl alcohol, povidone |
| Clear Eyes Redness Relief | glycerin, naphazoline HCL |
| Clear Eyes Tears Liquid Gel | carboxymethylcellulose, glycerin |
| Clear Eyes Tears Plus Redness Relief | glycerin, hydroxypropyl methylcellulose, naphazoline HCL |
| Clear Eyes Tears Plush Itchy Eye Relief | hydroxypropyl methylcellulose, polysorbate 80, naphazoline HCL, zinc sulfate |
| Clear Eyes Triple Action Relief | polyvinyl alcohol, povidone, tetrahydrozoline HCL |
| Clear View | tetrahydrozyline |
| Combigan | brimonidine tartrate, timolol maleate |
| Complete Blink-N-Clean | hydroxypropyl methylcellulose |
| Complete Lubricating and Rewetting drops | hydroxypropyl methylcellulose |
| Cortisporin | neomycin, polymixin |
| Cosopt | dorzolamide, timolol |
| Crolom | cromolyn |
| Crolom | cromolyn sodium |
| Cyclogyl | cyclopentolate |
| Dionephrine | phenylephrine |
| Diopentolate | cyclopentolate |
| Emadine | emedastine |
| Epicar | pilocarpine |
| Famciclovir | famciclovir |
| Flarex | fluorometholone |
| FML | fluorometholone |
| Garamycin | gentamycin |
| GenTeal | hydroxypropyl methylcellulose |
| GenTeal Lubricant Eye Gel | hydroxypropyl methylcellulose |
| GenTeal Mild | hydroxypropyl methylcellulose |
| GenTeal PF | hydroxypropyl methylcellulose |
| Goniovisc | hydroxypropyl methylcellulose |
| Homatropine | isopto homatropine |
| Hypotears | polyvinyl alcohol |
| I-Caps Eye Vitamins | multi vitamin |
| lopedine | apraclonidine |
| lopidine | apraclonidine |
| Isopto Atropine | atropine |
| Isopto Carpine | pilocarpine |
| Isopto Homatropine | homatropine |
| Isoptocarpine | pilocarpine |
| Istalol | timolol maleate |
| Lacrilube | mineral oil, white perolatum |
| Livostin | levocabastine |
| Llotycin | erythromycin |
| Lotemax | loteprednol |
| Lumigan | bimatoprost |
| Maxidex | dexamethasone |
| Maxitrol | dexamethasone, neomycin, polymixin |
| Mi-Pilo | pilocarpine |
| Mi-Pilo Ophth Sol | pilocarpine |
| Minims Pilocarpine | pilocarpine |
| Miocarpine | pilocarpine |
| Mistura P | pilocarpine |
| Murine Tears | polyvinyl alcohol, povidone |
| Mydfrin | phenylephrine |
| Mydrfrin | phenylephrine |
| Mydriacyl | tropicamide |
| Mydriacyl | tropicamide |
| Naphcon | naphazoline |
| Naphcon-A | naphazoline, pheniramine |
| Neosporin Ophthalmic | polymyxin B sulfate, neomycin sulfate, gramicidin |
| Nevanac | nepafenac |
| NutraTear | polyvinyl alcohol |
| Oasis Tears | glycerin |
| Oasis Tears Plus | glycerin |
| Ocu-Carpine | pilocarpine |
| Ocufen | flurbiprofen |
| Ocufen | flurbiprofen |
| Ocuflox | ofloxacin |
| OcuNefrin | phenylephrine |
| Ocupress | carteolol |
| Ocusert P 20 | pilocarpine |
| Ocusert Pilo | pilocarpine |
| Ocusert Pilo-20 | pilocarpine |
| Ocusert Pilo-40 | pilocarpine |
| Ocuvite | multi vitamin |
| Ocuvite Adult 50+ | multi vitamin |
| Ocuvite Lutein | multi vitamin |
| Omnipred | prednisolone |
| Ophthetic | proparacaine |
| Optivar | azelastine |
| Pataday | olopatadine |
| Patanol | olopatadine |
| Pilagan | pilocarpine |
| Piligan | pilocarpine |
| Pilocar | pilocarpine |
| Pilocar | pilocarpine |
| Pilocar SMP | pilocarpine |
| Pilocarpal | pilocarpine |
| Pilocarpin | pilocarpine |
| Pilocarpine chloride | pilocarpine |
| Pilocarpine HCl | pilocarpine |
| Pilocarpine monohydrochloride | pilocarpine |
| Pilocarpine muriate | pilocarpine |
| Pilocarpol | pilocarpine |
| Pilocel | pilocarpine |
| Pilokarpin | pilocarpine |
| Pilokarpin monohydrochloride | pilocarpine |
| | |
| Pilokarpol | pilocarpine |
| Pilomiotin | pilocarpine |
| Pilopine HS | pilocarpine |
| Piloptic-1 | pilocarpine |
| Piloptic-1/2 | pilocarpine |
| Piloptic-2 | pilocarpine |
| Piloptic-3 | pilocarpine |
| Piloptic-4 | pilocarpine |
| Piloptic-6 | pilocarpine |
| Pilostat | pilocarpine |
| Poly-Visc | parrafin |
| Polysporin | polymixin B, bacitracin |
| Polytrim | polymyxin B sulfate, trimethoprim |
| Pontocaine | tetracaine |
| Pred Forte | prednisone |
| Pred G | gentamicin, prednisolone |
| Prefrin Liquifilm | phenylephrine |
| PreserVision | multi vitamin |
| Proparacaine | proparacaine hydrochloride |
| Propine | dipivefrin |
| PV Carpine Liquifilm | pilocarpine |
| Refresh Optive | carboxymethylcellulose, glycerin |
| Refresh Optive Sensitive | carboxymethylcellulose, glycerin |
| Refresh Plus | carboxymethylcellulose |
| Refresh PM | mineral oil, petrolatum |
| Refresh Redness Relief | phenylephrine HCL, polyvinyl alcohol |
| Refresh Tears | carboxymethylcellulose |
| Rescula | unoprostone |
| Restasis | cyclosporine opthalmic emulsion |
| Rohto Arctic Eye Drops | hydroxypropyl methylcellulose, tetrahydrozoline hydrochloride |
| Rohto Cool Redness Relief | naphazoline Hydrochloride |
| Rohto Hydra | hydroxypropyl methylcellulose |
| Rohto Ice Redness Relief | hydroxypropyl methylcellulose, tetrahydrozoline hydrochloride |
| Salagen | pilocarpine |
| Similasan Computer Eyes | conium maculatum, natrium muriaticum, ruta graveolens |
| Similasan Pink Eye Relief | belladonna, euphrasia, hepar sulphuris |
| Sno Pilo | pilocarpine |
| Sochlor | sodium chloride |
| Soothe | glycerin |
| Soothe XP | light mineral oil, mineral oil |
| Soothe Xtra Hydration | glycerin, propylene glycol |
| Spersaphrine | phenylephrine |
| Sulamyd | sulfacetamide |
| Sulfacetamide | bleph-10, sulamyd |
| Syncarpine | pilocarpine |
| Systane | polyethylene glycol 400,polyethylene glycol |
| | |
| Systane Balance | propylene glycol |
| Systane Contacts | polyethylene glycol |
| Systane Free | polyethylene glycol 400, polyethylene glycol |
| Systane Nighttime | mineral oil, white petrolatum |
| Systane Preservative Free | polyethylene glycol |
| Systane Ultra | propylene glycol, polyethylene glycol |
| Systane Ultra Preservative Free | propylene glycol, polyethylene glycol |
| Tears Again | polyvinyl alcohol |
| Tears Again Liquid Gel Drops | carboxymethylcellulose |
| Tears Again MC | hydroxypropyl methylcellulose |
| Tears Again Night & Day Gel | carboxymethylcellulose |
| Tears Again Ointment | mineral Oil, White Petroleum |
| Tears Again Solution | polyvinyl alcohol |
| Tears Naturale Forte | hydroxypropyl methylcellulose |
| Tears Naturale Free | hydroxypropyl methylcellulose |
| Tears Naturale II | hydroxypropyl methylcellulose |
| Tears Naturale PM | mineral oil, white petrolatum |
| Thera Tears | carboxyl methylcellulose |
| Thera Tears Liquigel | carboxyl methylcellulose |
| Timoptic | timolol maleate |
| Timoptic Ocumeter | timolol maleate |
| Timoptic Ocumeter Plus | timolol maleate |
| Timoptic-XE | timolol maleate |
| Tobradex | tobramycin, dexamethasone |
| Tobrex | tobramycin, dexamethasone |
| Tozal | multi vitamin |
| Tropicacyl | tropicamide |
| Tropicamide | tropicamide |
| Trusopt | dorzolamide HCL |
| Valacyclovir | valacyclovir |
| Vancomycin | vancomycin |
| Vasocon | naphazoline |
| Vasocon-A | naphazoline, antazoline |
| Vexol | rimexolone |
| Vigamox | moxifloxacin HCL |
| Viroptic | trifluridine |
| Visine A | naphazoline HCL, pheniramine maleate |
| Visine A.C | tetrahydrozoline HCL, zinc sulfate |
| Visine Advanced Relief | dextran, polyethylene glycol, povidone, tetrahydrozoline HCL |
| Visine Contacts | hydroxypropyl methylcellulose, glycerin |
| Visine L.R. | oxymetazoline HCL |
| Visine Maximum Redness Relief | glycerin, hydroxypropyl ethylcellulose, polyethylene glycol, tetrahydrozoline |
| Visine Original | tetrahydrozoline HCL |
| Visine Tears | glycerin, hydroxypropyl ethylcellulose, polyethylene glycol |
| Visine Tears Long Lasting | glycerin, hydroxypropyl ethylcellulose, polyethylene glycol |
| Visine Tired Eye Relief | glycerin, hydroxypropyl ethylcellulose, polyethylene glycol |
| Visine Totality | glycerin, hydroxypropyl ethylcellulose,polyethylene glycol, tetrahydrozoline, zinc sulfate |
| Viteyes | multi vitamin |
| Voltaren | diclofenac |
| Xalatan | latanoprost |
| Xibrom | bromfenac |
| Zaditen | ketotifen fumarate |
| Zaditor | ketotifen fumarate |
| Zymar | gatifloxacin |

While the composition of the present invention has many advantages that all eye medications would benefit from, such as easier administration and increased contact time with the eye, particular benefit may be gained with medicines containing costly active ingredients. These types of costly medications will greatly benefit from the fact that much less active ingredient is necessary to produce the same result. The reduction in active ingredient is a result of a lower amount of medicament dispensed per application and increased corneal absorption from increased contact time. Some of the foaming agents listed above are currently excipients in extended release formulations, which reduce the number of applications required per day. This would further reduce the amount of medication needed.

While the previous written description of the invention and embodiments enables one of ordinary skill to make and use the present invention, those will understand and appreciate the existence of variations, combinations and equivalents of the specific embodiment, method, and examples herein. The invention should therefore not be limited by the embodiments, methods, and examples herein, but by all embodiments and methods within the scope and spirit of the invention as claimed.

The present invention is exemplified by the following examples, which are intended as purely illustrative and non-limiting.

### Example 1 - hydroxypropyl methyl cellulose as a foaming agent

This experiment demonstrates the ability of hydroxypropyl methyl cellulose to perform as a foaming agent.

Water (20 ml) was heated to 90°C. Next, 0.3 g hydroxypropyl methyl cellulose powder was added to the water with stirring. Once dispersed, 40 ml of cold water was added to the mixture with agitation. The mixture was allowed to cool for 30 minutes. The solution was then transferred into a foaming handsoap bottle and dispensed. That foam held its shape for 2 minutes and was mostly deflated after 7 minutes. The foam had an initial volume of 2 ml and a final volume of 0.2 ml, indicating that 90% of the foam volume was air. The solution viscosity was 200 mPas.

### Example 2 - hydroxypropyl methyl cellulose as a foaming agent in a composition including timolol

This experiment demonstrates the ability of hydroxypropyl methyl cellulose to perform as a foaming agent for a composition including a topical ophthalmic medicament.

A solution of 34 mg timolol maleate (0.25%) and 25 mg hydroxypropyl methylcellulose (0.5%) was made in 5 ml sterile saline solution (pH 6). The same technique from Example 1 was used to dissolve hydroxypropyl methyl cellulose in the saline solution. The solution was then transferred to a foaming handsoap bottle fitted with a micropipette tip. About 25 ml of solution was dispensed, producing a foam. Timolol activity was preserved.

### Examples 3 - bovine serum albumin as a foaming agent in a composition including timolol

Bovine serum albumin (250 mg) and timolol maleate (17 mg) were mixed with 5 ml of saline solution. When dispensed from a foaming handsoap bottle, a thick foam was produced with pH 7 and a viscosity of about 2 mPas.

## Claims

1. A liquid ophthalmic composition including a foaming agent, adapted for topical administration to the eye from an applicator capable of dispensing a foam.

2. A liquid ophthalmic composition as claimed in claim 1 wherein the composition further comprises of a therapeutically active ingredient.

3. A liquid ophthalmic composition as claimed in claim 1 or claim 2 wherein the foaming agent is selected from:
a carbohydrate selected from cellulose derivatives, chitosan, cyclodextrins, gums, quince seed extract, starch, chemically modified starches, and sugars;
a corneal penetration enhancer selected from surfactants including detergents and non-ionic surfactants.;
a gelling agent selected from gelatin and agar;
a glycosaminoglycan selected from hyaluronic acids and salts;
a lipid selected from cetyl alcohol, stearyl alcohol, and stearic acid;
a protein selected from an albumin and sodium caseinate;
a polyol selected from ethylene glycol, glycerol, and propylene glycol;
a synthetic polymer selected from amine-bearing polymers, carboxyvinyl polymers, polyacrylic acid, polyethylene glycol, polyvinyl acetate, polyvinyl alcohol, polyvinyl chloride, polyvinylidene chloride, and polyvinylpyrrolindone.

4. A liquid ophthalmic composition as claimed in claim 3, wherein the foaming agent is selected from a cellulose derivative, a corneal penetration enhancer, and an albumin.

5. A liquid ophthalmic composition as clamed in any one of the preceding claims, wherein the foaming agent is hydroxypropyl methylcellulose (hypromellose) in a concentration of between 0.25% and 5%.

6. A liquid ophthalmic composition as clamed in any one of claims 1-4, wherein the foaming agent is naturally derived or created albumin in a concentration of between 1% and 25%.

7. A liquid ophthalmic composition as claimed in any one of the preceding clams, wherein the therapeutically active agent comprises one or more of:
acyclovir,antazoline,apraclonidine,atropine,azelastine,bacitracin, bepotastine besilate, betaxolol, bimatoprost, brimonidine tartrate, brinzolamide, bromfenac, carboxymethylcellulose, carteolol hydrochloride, ciprofloxacin hydrochloride, conium maculatum, cromolyn sodium, cyclopentolate, cyclosporine, dexamethasone, dextran, diclofenac, dipivefrin hydrochloride, dorzolamide hydrochloride, emedastine, erythromycin, euphrasia, famciclovir, fluorometholone, flurbiprofen, gatifloxacin, gentamicin, glycerin, gramicidin, hepar sulphuris calcareum, homatropine, hydroxypropylmethylcellulose, isoptoatropine sulfate, isoptohomatropine hydrobromide, ketorolac, ketotifenfumarate, latanoprost, levobunolol hydrochloride, levocabastine hydrochloride light mineral oil (also called mineral oil, paraffin), lodoxamide tromethamine, loteprednol etabonate, moxifloxacin hydrochloride, multivitamin, N-Acetyl-L-Carnosine, naphazoline, naphazoline hydrochloride, naphazolineHydrochloride, natrium muriaticum, nedocromil sodium sulfate, neomycin, nepafenac, norfloxacin, ofloxacin, olopatadine hydrochloride, oxymetazoline hydrochloride, pectin, pemirolast potassium, petrolatum (also called white petrolatum, white petroleum), pheniramine maleate, phenylephrine hydrochloride, pilocarpine hydrochloride, polyethyleneglycol (for example,polyethylene glycol 400), polymixin, polymyxin B sulfate, polysorbate 80, polyvinyl alcohol (PVA), povidone, povidone-iodine, prednisolone, prednisone,proparacaine hydrochloride, propyleneglycol, rimexolone, ruta graveolens, sodium chloride, sufacetamide sodium, soy lecithin, tetracaine, tetrahydrozoline hydrochloride, tetrahydrozoline hydrochloride, timolol maleate, tobramycin, trifluridine, trimethoprim, tropicamide, unoprostone isoproppyl, valacyclovir (also called valaciclovir), vacomycin, and zinc sulphate; or from a recombinant DNA, antibody, protein, or biopharmaceutical for medicinal purposes.

8. A liquid ophthalmic composition as claimed in claim 7, wherein the therapeutically active agent is timolol malate.

9. A liquid ophthalmic composition as claimed in any one of the preceding claims, wherein said composition does not include a propellant.

10. A topical ophthalmic composition delivery system comprising a liquid ophthalmic composition as claimed in any one of claims 1-9, and an applicator capable of dispensing a foam.

11. A topical ophthalmic composition delivery system as claimed in claim 10, wherein said liquid ophthalmic composition is contained in said applicator, and forms a foam when mixed with a gas.

12. A topical ophthalmic composition delivery system as claimed in claim 11, wherein said gas is or contains a therapeutically active agent.

13. A topical ophthalmic composition delivery system as claimed in claim 12, wherein said composition does not include a therapeutically active agent and the therapeutically active agent is contained only in said gas.

14. A topical ophthalmic composition delivery system as claimed in any one of claims 10 to 13, wherein the container is a foam dispensing pump container.

15. An ophthalmic foam containing a therapeutically active agent and a foaming agent.
